# EUROPÄISCHE PATENTSCHRIFT

(11) **EP 0 894 477 B1**
(45) Veröffentlichungstag und Bekanntmachung des Hinweises auf die Patenterteilung: **10.01.2001**
(21) Anmeldenummer: 98116343.9
(22) Anmeldetag: 28.08.1998
(51) Int. Cl.: A61B 17/70

(54) **Bridenteil zum Einspannen einer Fixierstange, und diesen aufweisende Haken und Stangenverbinder**
Fixation rod clamp, and hook and rod connector incorporating the same
Bride de serrage d'une tige de fixation, et crochet et raccord de tiges incorporants cette bride

(30) Priorität: 06.10.1997 CH 233397
(43) Veröffentlichungstag der Anmeldung: 03.02.1999
(73) Patentinhaber: Hermann, Werner, CH-6312 Steinhausen (CH)
(72) Erfinder: Hermann, Werner, 6312 Steinhausen (CH); Matzen, Klaus A., Prof. Dr. Dr., 86199 Augsburg-Göggingen (DE); Buetler, Beat, 6331 Hünenberg (CH)
(74) Vertreter: Hotz, Klaus, Dipl.-El.-Ing./ETH

(56) Entgegenhaltungen:
- EP-A- 0 536 066
- WO-A-95/28889
- WO-A-98/44859
- DE-A- 3 924 050
- US-A- 4 483 334
- US-A- 5 222 954
- US-A- 5 613 968

## Beschreibung

Die Erfindung betrifft einen Bridenteil zum Einspannen einer Fixierstange nach dem Oberbegriff des Anspruchs **1**, einen Haken mit einem Bridenteil nach dem Oberbegriff des Anspruchs **7** und einen Stangenverbinder mit einem Bridenteil nach dem Oberbegriff des Anspruchs **9**.

Haken und Stangenverbinder dieser Art werden in der Orthopädie für Patienten verwendet, die beispielsweise wegen Spondilose temporär oder definitiv eine interne Fixateureinrichtung benötigen. Unter dem Begriff Haken sollen im Rahmen der vorliegenden Beschreibung sämtliche derartigen Haken wie Pedikelhaken, Laminahaken, Lumbalhaken und Transversalhaken verstanden werden, wobei die letzteren als Sicherungshaken, z.B. für Pedikelhaken, verwendet werden. Mit einer internen Fixateureinrichtung wird angestrebt, dass der Patient beschwerdefrei wird, auch wenn dabei in Kauf genommen werden muss, dass seine Beweglichkeit eingeschränkt wird. Im allgemeinen wird pro Körperseite eine im wesentlichen in Richtung der Wirbelsäule verlaufende Fixierstange vorgesehen, wobei jede der Fixierstangen mit mindestens zwei Haken bestückt ist. Die beiden Fixierstangen werden durch etwa horizontal verlaufende Querstangen, die auch als Querverbinder bezeichnet werden, miteinander gekoppelt, wobei jeweils zur gegenseitigen Befestigung von Fixierstange und Querstange ein Stangenverbinder benutzt wird.

Die Haken weisen einen eigentlichen Hakenteil, welcher von hinten eine sich seitlich erstreckende Partie der Wirbelsäule unter- oder übergreift, und einen integral mit dem Hakenteil hergestellten Bridenteil auf, welcher zur Aufnahme und Befestigung der Fixierstange dient.

Die Stangenverbinder weisen eine Einspannvorrichtung zur Befestigung an der Querstange und einen Bridenteil zum Einspannen der Fixierstange auf, wobei die Einspannvorrichtung für die Querstange gleich oder anders ausgebildet sein kann wie der Bridenteil für die Fixierstange.

Der Bridenteil, der beim Haken und beim Stangenverbinder im wesentlichen gleich ausgebildet ist, bildet eine in Richtung der Fixierstange verlaufende Rinne, mit einem Rinnengrund und zwei seitlich an diesen anschliessenden Seitenwandungen. Beim Anbringen der internen Fixateureinrichtung wird beispielsweise so vorgegangen, dass pro Körperseite die erforderlichen Haken angebracht werden, dann die Fixierstange mit den Stangenverbindern seitlich in die durch den Bridenteil gebildete Rinne eingeführt wird, daraufhin die Querstangen angebracht und fixiert werden und schliesslich mittels Schraubenverbindungen die Haken und Stangenverbinder an der Fixierstange definitiv befestigt werden.

Für die einwandfreie Wirkung eine solchen internen Fixateureinrichtung ist eine perfekte gegenseitige Verbindung zwischen den Haken bzw. den Stangenverbindern und der Fixierstange von ausschlaggebender Bedeutung. Diese Verbindung darf sich weder durch Wärmeeinwirkungen noch durch mechanische Beanspruchung lockern, und das Entstehen von Abriebpartikel muss vermieden werden.

Bei einem bekannten Haken ist der Bridenteil so ausgebildet, dass man eine Schraube in eine plan ausgebildete Seitenwandung der Rinne einschraubt, wodurch die Front des Schraubenbolzens auf die Fixierstange gepresst wird, so dass die Fixierstange zwischen dem Schraubenbolzen und der gegenüberliegenden Seitenwandung eingeklemmt bzw. eingespannt ist. Es liegt auf der Hand, dass eine solche Befestigung unbefriedigend ist. Einerseits kann sich die Fixierstange verhältnismässig leicht quer zu ihrer Längsachse entlang der planen Seitenwand verschieben und anderseits wird am Ort der praktisch nur punktuellen Berührung zwischen Schraubenbolzen und Fixierstange ein sehr hoher Druck ausgeübt, was die Entstehung von Abriebpartikeln begünstigt.

Bei einem weiteren bekannten Haken, dessen Bridenteil dem eingangs genannten Stand der Technik entspricht, bildet dieser Bridenteil eine Rinne mit einem im Schnitt halbkreisförmigen Rinnengrund, welcher sich beidseitig in zueinander parallelen Seitenwandungen fortsetzt. Die Befestigung erfolgt auch hier durch eine Schraube, welche auf die Oberfläche der Fixierstange wirkt. Die Verlängerung der Schraubenlängsachse verläuft nicht radial durch den entsprechenden Querschnitt der Fixierstange sondern in Richtung einer Sekante dieses Querschnittes, und zwar im Bereich derjenigen Querschnittshälfte, die nicht am Rinnengrund anliegt. Dadurch wird zwar eine Verbesserung gegenüber dem zuerst geschilderten vorbekannten Haken erreicht, weil durch die bezüglich der Fixierstange exzentrisch angeordnete Schraube eine Verschiebung der Fixierstange quer zu ihrer Längsrichtung verhindert wird. Auch bei diesem Haken erfolgt aber, wie schon erwähnt, die Befestigung der Fixierstange im Befestigungsteil mittels einer Schraube, deren Front direkt auf die Fixierstange gepresst wird, was, wie bekannt, zur Entstehung von Abriebpartikeln führen kann.

Ferner ist aus der **EP-A1-0 536 066** ein Bridenteil bekannt, welcher eine Fixierstange um mehr als 180° umgibt. Der Bridenteil ist zweiteilig und weist ein unteres und ein oberes Bridenelement auf. Das untere Bridenelement bildet eine Aufnahme für die Fixierstange und umgibt diese über mehr als 180°. Das obere Bridenelement überfängt das untere Bridenelement und wird mittels Schrauben auf das untere Bridenelement vorgespannt; die Innenflächen des oberen Bridenelementes gelangen hierbei zur Anlage an den Aussenflächen des unteren Bridenelementes und drücken diese, dank keilförmiger Ausbildung, um so mehr zusammen, je stärker die Schrauben angezogen werden. Nachteilig an dieser Einrichtung ist die zweiteilige Ausbildung des Bridenteils.

Im weiteren ist aus der **US-4,483,333** ein Bridenteil der eingangs genannten Art bekannt. mit einer Rinne, welche die Fixierstange um mehr als 180° umschliesst und welche sich in zwei Seitenwandungen fortsetzt, die mittels einer Schraubeneinrichtung zusammengespannt werden. Der Bridenteil umgibt die Stange nahezu über ihren gesamten Umfang. Dies hat zur Folge, dass eine einwandfreie Führung der Fixierstange gegen Verschiebungen quer zu ihrer Achse erreicht wird. Nachteilig ist aber, dass zum, Einführen der Fixierstange die Seitenwandungen beträchtlich auseinandergespreizt werden müssen.

Zusammenfassend kann festgestellt werden, dass keine Bridenteile für Haken und Stangenverbinder bekannt sind, die so ausgebildet sind, dass sie bei einfacher Montage eine in jeder Beziehung einwandfreie Verbindung mit der Fixierstange gewährleisten.

Die Aufgaben der Erfindung werden somit darin gesehen,
- den Bridenteil der eingangs genannten Art in der Weise zu verbessern, dass die erwähnten Nachteile vermieden werden, und ferner
- einen verbesserten Haken sowie
- einen verbesserten Stangenverbinder vorzuschlagen.

Diese Aufgaben werden erfindungsgemäss durch die Merkmale der kennzeichnenden Teile der unabhängigen Ansprüche **1**, **7** und **9** gelöst. Die Ansprüche **2** bis **6** definieren bevorzugte Ausbildungsformen des erfindungsgemässen Bridenteils, der Anspruch **8** eine Einzelheit des erfindungsgemässen Hakens und der Anspruch **10** eine bevorzugte Ausbildung der Stangenverbinders. .

Der neue Bridenteil ist so ausgebildet, dass er eine Rinne bildet, deren Rinnengrund sich im Schnitt nicht nur über einen Winkel von 180° sondern noch über einen zusätzlichen kleinen Teilwinkel erstreckt, so dass die Fixierstange über mehr als die Hälfte ihres Umfanges vom Rinnengrund, jedoch nicht über nahezu ihren gesamten Umfang umschlossen wird. Zum Einführen der Fixierstange müssen daher die Seitenwandungen des Bridenteils nicht übermässig gespreizt werden. Das Einspannen der Fixierstange im Bridenteil mittels der Schraubeneinrichtung erfolgt wie üblich durch das Zusammenspannen der beiden Seitenwandungen der Rinne. Dabei wird erstens eine Verschiebung der Fixierstange quer zu ihrer Achse durch die neue Form der Rinne verhindert, und zweitens wirkt die beim Einspannen der Fixierstange ausgeübte Kraft längs einer beträchtlichen Fläche, so dass hohe punktuelle Belastungen vermieden werden. Als Hilfe bei der Montage wird der Abstand der Gewindelängsachse vom Zentrum des dem Rinnengrund einschreibbaren Kreises bzw. von der Längsachse der Fixierstange so festgelegt, dass er der Summe der Radien des erwähnten einschreibbaren Kreises und des Schraubenbolzens entspricht, welche letzterer vorzugsweise in diesem Bereich gewindefrei ist. Dadurch wirkt die Fixierstange beim Beginn des Einschraubens der Schraube als Führung für diese und im montierten Zustand liegt der Schraubenbolzen an der Fixierstange an.

Im allgemeinen wird der Bridenteil derart konstruiert, dass der gegenseitige Abstand der Seitenwandungen etwas geringer ist als der Durchmesser der Rinne bzw. der Fixierstange, so dass sich die Seitenwandungen direkt an die Öffnung des Rinnengrundes anschliessen. Es wäre aber auch möglich, den gegenseitigen Abstand der Seitenwandungen entsprechend dem Durchmesser des Rinnengrundes bzw. der Fixierstange zu wählen, wobei die Seitenwandungen dann in einer geringen Distanz von der Öffnung des Rinnengrundes beginnen würden.

Die Schraubeneinrichtung ist vorzugsweise so ausgebildet, dass die eine der Seitenwandungen der Rinne ein Innengewinde und die andere der Seitenwandungen der Rinne eine Durchgangsbohrung aufweist. Zum Zusammenspannen der beiden Seitenwandungen wird durch die Durchgangsbohrung in das Innengewinde eine Kopfschraube oder ein Schraubenbolzen mit einer Mutter eingeschraubt.

Bei der Festlegung der Einschraubtiefe bzw. der Anzahl der eingeschraubten Gewindegänge sind zwei Erfordernisse zu berücksichtigen: einerseits soll der Bridenteil möglichst wenig Volumen aufweisen, um muskuläre Probleme beim Patienten zu minimalisieren; anderseits muss eine bestimmte Einschraubtiefe vorhanden sein, um eine genügende Starrheit der Schraubenverbindung zu erzielen. Es hat sich daher als günstig erwiesen, die mit dem Innengewinde versehene Seitenwandung in Richtung der Gewindelängsachse stärker auszubilden als die mit der Durchgangsbohrung versehene Seitenwandung.

Um die Schraubenverbindung auch ohne Verwendung eines zusätzlichen Sicherungselementes zu sichern, kann die Aussenfläche der mit der Durchgangsbohrung versehenen Seitenwandung, an welcher im montierten Zustand der Schraubenkopf der Kopfschraube oder die Mutter anliegen, einen oder gegebenenfalls mehrere kleine Vorsprünge aufweisen, die beim Anziehen der Schraubenverbindung zusammengedrückt werden und daher einen elastischen Gegendruck auf den Schraubenkopf oder die Mutter ausüben, so dass die Schraubenverbindung gegen Lösen gesichert ist.

Der erfindungsgemässe Haken weist neben einem Bridenteil nach der Erfindung einen eigentlichen Hakenteil auf, der zum Unter- oder Übergreifen einer sich im wesentlichen seitlich erstreckenden Partie einer Wirbelsäule eines Patienten bestimmt ist.

Je nach Verwendung des Hakens wird der eigentliche Hakenteil so ausgebildet, dass der Haken ein Pedikel-,. Lamina-, Lumbal- oder Transversalhaken ist, und von allen Haken werden sowohl rechtsseitig wie auch linksseitig verwendbare Ausführungen in gerade wie auch in abgewinkelten Varianten hergestellt.

Der erfindungsgemässe Stangenverbinder weist neben einem Bridenteil nach der Erfindung eine Einspannvorrichtung für eine Querstange auf.

Der Haken bzw. der Stangenverbinder werden erfindungsgemäss zusammen mit einer Fixierstange, an welcher sie mittels der Schraubeneinrichtung befestigt sind, als Teil einer orthopädischen Fixateureinrichtung verwendet, wobei die Seitenwandungen des Bridenteils, deren Abstand im allgemeinen etwas geringer ist als der Durchmesser der Fixierstange, vor dem Einbringen der Fixierstange elastisch voneinander weggespreizt werden.

Beim Festziehen der Schraubeneinrichtung werden vorzugsweise die Seitenwandungen elastisch und gegebenenfalls auch plastisch deformiert.

Zur Vermeidung der Bildung von Spannungselementen werden vorzugsweise alle Bestandteile der internen Fixateureinrichtung, also die Haken, die Schraubeneinrichtung und die Fixierstange, aus identischem Material hergestellt, wobei sich als besonders günstiges Material Titan-Niob-Legierungen erwiesen haben.

Weitere Eigenschaften und Vorteile der Erfindung werden im folgenden anhand von Ausführungsbeispielen und mit Bezug aus die Zeichnung ausführlich beschrieben. Es zeigt:
- **Fig. 1**: einen Teil einer internen Fixateureinrichtung mit einem als Pedikelhaken ausgebildeten Haken nach der Erfindung, in einem Schaubild;
- **Fig. 2**: einen weiteren Haken nach der Erfindung, in einer Ansicht von der Seite;
- **Fig. 3**: den in **Fig. 2** dargestellten Haken, in einer Ansicht von oben;
- **Fig. 4**: einen Stangenverbinder nach der Erfindung, in einer Ansicht von oben;
- **Fig. 5**: den in **Fig. 4** dargestellten Stangenverbinder, in einer Ansicht von der Seite;
- **Fig. 6**: einen Abschnitt einer Wirbelsäule mit interner FixateurEinrichtung, mit zwei Fixierstangen und mehreren Haken, in einer Ansicht von hinten; und
- **Fig. 7**: den in **Fig. 6** dargestellten Abschnitt einer Wirbelsäule mit interner Fixateureinrichtung, in einer Ansicht von der Seite.

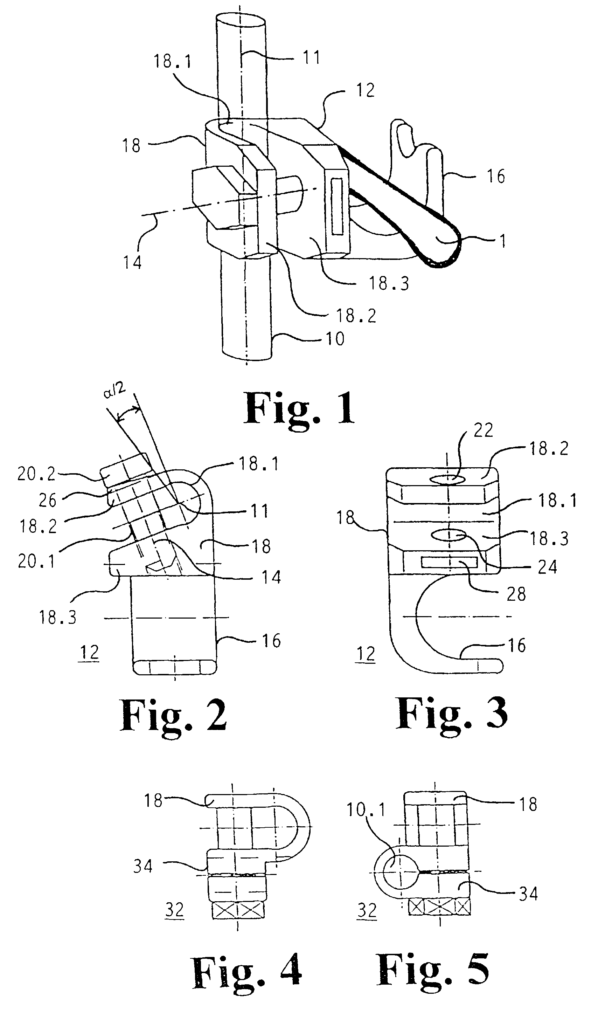

**Fig. 1** zeigt stark vereinfacht eine sich seitwärts erstreckende Partie **1** einer Wirbelsäule eines menschlichen Patienten mit einem Abschnitt einer internen Fixateureinrichtung, umfassend eine Fixierstange **10** und ein Haken **12** mit einer Schraubeneinrichtung mit der Achse **14**. Der Haken **12** weist einen eigentlichen Hakenteil **16** und einen Bridenteil **18** auf, welcher im vorliegenden Fall die Partie **1** der Wirbelsäule in **Fig. 1** von links nach rechts - vom Patienten aus gesehen von hinten nach vorn - untergreift. Der Bridenteil **18** dient dazu, die Fixierstange **10** und den Haken **12** aneinander zu befestigen. Der Bridenteil **18** bildet eine Rinne mit einem Rinnengrund **18.1**, an welchem die Fixierstange **10** anliegt, und zwei an den Rinnengrund **18.1** anschliessende Seitenwandungen **18.2**, **18.3**. Mittels der Schraubeneinrichtung mit der Achse **14** werden die beiden Seitenwandungen **18.2**, **18.3** des Bridenteils **18** zusammengespannt, wodurch die Fixierstange **10** an ihrem im Bridenteil **18** aufgenommenen Bereich festgeklemmt wird. Der Haken **12** nimmt im implantierten Zustand eine Stellung ein, bei der seine Öffnung, durch welche die Fixierstange **10** eingeführt wird, seitlich gerichtet ist, und das Anziehen der Schraubeneinrichtung erfolgt in **Fig. 1** gesehen von links - vom Patienten aus gesehen also von hinten -. Der in **Fig. 1** dargestellte Haken **12** ist somit zur Verwendung auf der rechten Körperseite und, wie schon erwähnt, zum Untergreifen der Partie 1 der Wirbelsäule ausgebildet. Die Grösse und Ausbildung des Hakens hängt von der vorgesehenen Verwendung und von der Körpergrösse des Patienten ab.

In den **Fig. 2** und **3** ist ein Haken **12** dargestellt, der in dieser Form im Massstab 1:1 hergestellt und verwendet werden könnte. In **Fig. 2** ist ausserdem eine Schraube der Schraubeneinrichtung mit einem Schraubenbolzen **20.1** und einer Mutter bzw. einem Schraubenkopf **20.2** dargestellt. Wie beim Haken gemäss **Fig. 1** weist auch dieser Haken **12** den Hakenteil **16** und den Bridenteil **18** auf. Der Bridenteil **18** bildet eine Rinne mit dem Rinnengrund **18.1** und den zwei Seitenwandungen **18.2, 18.3**.

Der Rinnengrund **18.1** ist, wie in **Fig. 3** sichtbar, so ausgebildet, dass er die in den **Fig. 2** und **Fig. 3** nicht dargestellte Fixierstange längs mehr als 180°, nämlich um 180° plus den zusätzlichen Teilwinkel α, genauer um 180° + 2 α/2, umgibt. Dadurch ist bei einer Konfiguration gemäss **Fig. 2** der gegenseitige Abstand der beiden Seitenwandungen **18.2, 18.3** etwas geringer als der Durchmesser des dem Rinnengrund **18.1** einschreibbaren Kreises bzw. der Fixierstange **10**. Zum Einführen der Fixierstange **10** müssen daher die Seitenwandungen **18.2, 18.3** leicht auseinandergespreizt werden.

Wie insbesondere aus **Fig. 3** ersichtlich, sind in der Seitenwandung **18.2** eine Durchgangsbohrung **22** und in der Seitenwandung **12.3** eine Bohrung bzw. ein Innengewinde **24** angebracht. Um eine genügende Gewindelänge zu erhalten, ist die Seitenwandung **18.3** in Richtung der Achse **14** stärker ausgebildet als die Seitenwandung **18.2**.

Beim Anziehen der Schraube **20.1, 20.2** erfolgt eine elastische und gegebenenfalls auch eine plastische Deformation der beiden Seitenwandungen **18.2, 18.3,** insbesondere natürlich der schwächeren Seitenwandung **18.2**.

**Fig. 2** zeigt, dass der Abstand der Achse **14** der Schraubeneinrichtung vom Zentrum des dem Rinnengrund **18.1** einschreibbaren Kreises bzw. von der Längsachse **11** der Fixierstange **10** und der Durchmesser des Schraubenbolzens **20.1** so bemessen sind, dass sich im montierten Zustand der Schraubenbolzen **20.1** und die Fixierstange **10** berühren.

Die zum Äusseren des Bridenteils - bzw. beim Patienten nach hinten - gewandte Fläche der Seitenwandung **18.2** weist einen Vorsprung **26** auf, der beim Anziehen der Schraube durch die Mutter **20.2** elastisch deformiert wird und dadurch eine Schraubensicherung bildet.

Eine Ausnehmung **28** dient zum Ergreifen des Hakens mit einer geeigneten Zange. Die Ausnehmung **28** und/oder weitere Ausnehmungen zum gleichen Zwecke können auch andernorts am Haken **12** angeordnet sein.

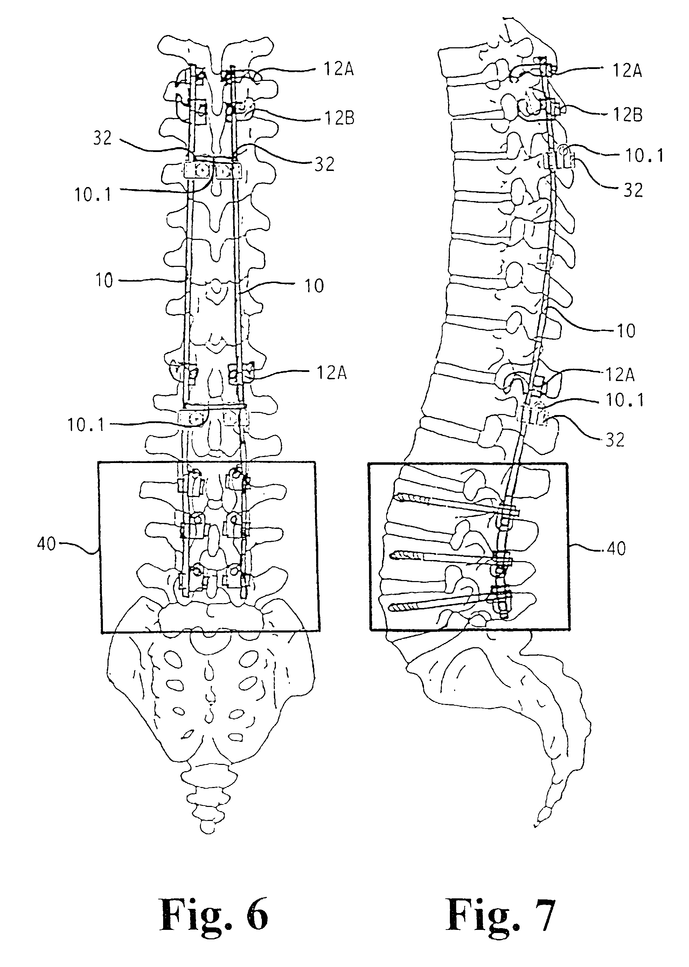

Die **Fig. 4** und **5** zeigen einen Stangenverbinder **32**, welcher zur gegenseitigen Verbindung der im wesentlichen vertikalen Fixierstange **10** mit einer mindestens annähernd horizontalen Querstange **10.1** dient, wie dies aus **Fig. 6** ersichtlich ist. Der Stangenverbinder **32** weist eine Einspannvorrichtung **34** für die Querstange **10.1** und den Bridenteil **18** zum Einspannen der Fixierstange **10** auf, welcher im wesentlichen gleich ausgebildet ist wie der im Zusammenhang mit den Haken der **Fig. 2** und **3** beschriebene Bridenteil.

In den **Fig. 6** und **7** ist ein Abschnitt der Wirbelsäule des Patienten mit einer internen Fixateureinrichtung dargestellt. Die letztere besteht aus zwei Fixierstangen **10**, von denen jede mit mehreren über- und untergreifenden Haken **12** bestückt ist. Zwischen den beiden Fixierstangen **10** verlaufen, wie üblich, Querstangen **10.1**, wobei die Fixierstangen **10** und die Querstangen **10.1** mittels Stangenverbindern **32** aneinander befestigt sind. Die Haken und insbesondere die Hakenteile sind je nach ihrer Verwendung ausgebildet. beispielsweise als Pedikel-, Laminar-, Lumbal oder Transversalhaken ausgebildet. In den **Fig. 6** und **7** sind Pedikelhaken mit **12A** und Transversalhaken mit **12B** bezeichnet. Mit 40 ist der Bereich bezeichnet, in welchem die Verankerung der Fixateureinrichtung beispielsweise mittels Pedikelschrauben erfolgt.

Haken, Schrauben, Fixierstangen, Querstangen und alle weiteren Implantatbestandteile sind aus demselben Material, vorzugsweise aus einer geeigneten Titan-Niob-Legierung, hergestellt.

Neben den in den Fig. 1 bis 7 dargestellten und oben beschriebenen Haken bzw. Stangenverbindem sind im Rahmen der Erfindung eine Vielzahl weiterer Ausführungsformen möglich.

## Patentansprüche

1. Bridenteil (**18**) zum Einspannen einer Fixierstange (**10**), welcher eine Rinne mit einem sich im Schnitt längs eines Winkels von 180° und eines zusätzlichen Teilwinkels (**a**) erstreckenden Rinnengrund (**18.1**) aufweist und zwei sich an den Rinnengrund (**18.1**) anschliessende Seitenwandungen (**18.2, 18.3**) besitzt, wobei der Rinnengrund (**18.1**) zur längsverschieblichen Aufnahme der an ihm anliegenden und von ihm längs mehr als 180° umschliessbaren, im Wesentlichen längs einer Wirbelsäule verlaufenden Fixierstange (**10**) bestimmt ist, und wobei zum Einspannen der Fixierstange (**10**) eine Schraubeneinrichtung (**20.1, 20.2**) mit einem Schraubenbolzen (**20.1**) und einer Gewindelängsachse (**14**) vorgesehen ist, welche Schraubeneinrichtung (**20.1, 20.2**) so ausgebildet und angeordnet ist, dass die Seitenwandungen (**18.2, 18.3**) des Bridenteils (**18**) zueinander gedrückt werden, um die Fixierstange (**10**) einzuspannen,
**dadurch gekennzeichnet,**
dass der Abstand der Gewindelängsachse (**14**) vom Zentrum des dem Rinnengrund (**18.1**) einschreibbaren Kreises gleich der Summe der Radien dieses einschreibbbaren Kreises und des vorzugsweise in diesem Bereich gewindefreien Schraubenbolzens (**20.1**) ist.

2. Bridenteil (**18**) nach Anspruch **1**,
**dadurch gekennzeichnet,**
dass der gegenseitige Abstand der Seitenwandungen (**18,2, 18.3**) kleiner ist als der Durchmesser des dem Bridengrund (**18.1**) einschreibbaren Kreis.

3. Bridenteil (**18**) nach mindestens einem der obigen Ansprüche,
**dadurch gekennzeichnet,**
dass die Schraubeneinrichtung (**20.1, 20.2**) ein Innengewinde (**24**) in einer der Seitenwandungen (**18.3**) und ein Aussengewinde an einem durch eine Durchgangsbohrung (**22**) in der der anderen Seitenwandung (**18.2**) in das Innengewinde (**24**) einschraubbaren Schraubenbolzen (**20.1**) aufweist.

4. Bridenteil (**18**) nach mindestens einem der obigen Ansprüche,
**dadurch gekennzeichnet,**
dass die mit dem Innengewinde (**24**) versehene Seitenwandung (**18.3**) in Richtung der Achse (**14**) der Schraubeneinrichtung (**20.1, 20.2**) stärker ausgebildet ist als die mit der Durchgangsbohrung (**22**) versehene Seitenwandung (**18.2**).

5. Bridenteil (**18**) nach mindestens einem der obigen Ansprüche,
**dadurch gekennzeichnet,**
dass die zum Äusseren des Bridenteils (**18**) gewandte Fläche der mit der Durchgangsbohrung (**22**) versehenen Seitenwandung (**18.2**) mindestens einen Vorsprung (**26**) aufweist, welcher mittels einer Mutter (**20.2**) bzw. einem Schraubenkopf der Schraubeneinrichtung (**20.1, 20.2**) elastisch deformierbar ist, um eine Schraubensicherung zu bilden.

6. Bridenteil (**18**) nach mindestens einem der obigen Ansprüche,
**dadurch gekennzeichnet,**
dass die Seitenwandungen (**18.2, 18.3**) des Bridenteils (**18**), deren gegenseitiger Abstand kleiner ist als der Durchmesser der Fixierstange (**10**), vor dem Einbringen der Fixierstange (**10**) elastisch voneinander wegspreizbar sind.

7. Haken (**12**) mit einem Bridenteil (**18**) zum Einspannen einer Fixierstange (**10**) und mit einem mit dem Bridenteil (**18**) integralen, von einer Seitenwandung (**18.3**) des Bridenteils (**18**) wegragenden Hakenteil (**16**), der zum Über- oder Untergreifen einer sich seitlich erstreckenden Partie (**1**) einer Wirbelsäule bestimmt ist,
**dadurch gekennzeichnet,**
dass der Bridenteil (**18**) nach mindestens einem der Ansprüche **1** bis **6** ausgebildet ist.

8. Haken (**12**) nach Anspruch **6**,
**dadurch gekennzeichnet,**
dass der Hakenteil (**16**) so ausgebildet ist, dass der Haken (**12**) als Pedikel-, Lamina-, Lumbal- oder Transversalhaken verwendbar ist.

9. Stangenverbinder (**32**) mit einen Bridenteil (**18**) zum Einspannen einer Fixierstange (**10**) und mit einer mit dem Bridenteil (**18**) integralen Einspannvorrichtung (**34**) für eine quer zur Fixierstange verlaufende Querstange (**10.1**),
**dadurch gekennzeichnet,**
dass der Bridenteil (**18**) nach mindestens einem der Ansprüche **1** bis **6** ausgebildet ist.

10. Stangenverbinder nach Anspruch **9**,
**dadurch gekennzeichnet,**
dass der Stangenverbinder **32** einschliesslich der Schraubeneinrichtung, (**20.1, 20.2**) aus identischem Material, vorzugsweise aus einer Titan-Niob-Legierung, hergestellt sind wie die weiteren Bestandteile der Fixateureinrichtung.

## Claims

1. Shackle component (18) for clamping a fixation rod (10), having a channel with a channel base (18.1) that in section extends along an angle of 180° plus an additional angle increment (a) and having two side walls (18.2, 18.3) adjoining the channel base (18.1), the channel base (18.1) being intended for receiving the fixation rod (10), which essentially extends alongside a spinal column, with the fixation rod (10) abutting against the channel base (18.1) so as to be longitudinally displaceable in it, and so that the fixation rod (10) can be surrounded by the channel base (18.1) by more than 180°, and a screw arrangement (20.1, 20.2) for clamping the fixation rod (10) being provided having a part (20.1) that has an outer thread and a longitudinal screw thread axis (14), the screw arrangement (20.1, 20.2) being so constructed and arranged that the side walls (18.2, 18.3) of the shackle component (18) are pressed towards each other to clamp the fixation rod (10), characterised in that the distance of the longitudinal screw thread axis (14) from the centre of the circle inscribable in the channel base (18.1) equals the sum of the radii of this inscribable circle and of the threaded bolt (20.1) which in this region is preferably unthreaded.

2. Shackle component (18) according to claim 1, characterised in that the distance apart of the side walls (18.2, 18.3) is smaller than the diameter of the circle inscribable in the shackle base (18.1).

3. Shackle component (18) according to at least one of the above claims, characterised in that the screw arrangement (20.1, 20.2) has an inner thread (24) in one of the side walls (18.3) and an outer thread on a threaded bolt (20.1) which can be screwed into the inner thread (24) via a through bore (22) in the other side wall (18.2).

4. Shackle component (18) according to at least one of the above claims, characterised in that the side wall (18.3) that has the inner thread (24) is of thicker construction seen in the direction of the axis (14) of the screw arrangement (20.1, 20.2) than the side wall (18.2) that has the through bore (22).

5. Shackle component (18) according to at least one of the above claims, characterised in that the surface of the side wall (18.2) that has the through bore (22), namely the surface facing outwards from the shackle component (18), has at least one projection (26) which is elastically deformable by a nut (20.2) or a screw head of the screw arrangement(20.1, 20.2) to lock the screw.

6. Shackle component (18) according to at least one of the above claims, characterised in that the side walls (18.2, 18.3) of the shackle component (18) whose distance apart is smaller than the diameter of the fixation rod (10) can be elastically splayed apart before the fixation rod (10) is introduced.

7. Hook (12) having a shackle component (18) for clamping a fixation rod (10), and having a hook component (16) integral with the shackle component (18) jutting out from a side wall (18.3) of the shackle component (18) and intended to engage over or under a laterally extending part (1) of a spinal column, characterised in that the shackle component (18) is constructed according to at least one of claims 1 to 6.

8. Hook (12) according to claim 6, characterised in that the hook component (16) is constructed so that the hook (12) can be used as a pedicle hook, lamina hook, lumbar hook or transverse process hook.

9. Rod connector (32) having a shackle component (18) for clamping a fixation rod (10) and having a clamping device (34) integral with the shackle component (18) for a transverse rod (10.1) extending transversely with respect to the fixation rod, characterised in that the shackle component (18) is constructed according to at least one of claims 1 to 6.

10. Rod connector according to claim 9, characterised in that the rod connector 32, including the screw arrangement (20.1, 20.2), are made from identical material as the other components of the fixation arrangement, preferably a titanium niobium alloy.

## Revendications

1. Elément de bride (18) permettant de serrer une tige de fixation (10), qui présente une gouttière avec une base de gouttière (18.1) s'étendant en coupe le long d'un angle de 180° et d'un pas angulaire supplémentaire (a) et possède deux parois latérales (18.2, 18.3) se raccordant à la base de gouttière (18.1), la base de gouttière (18.1) étant définie pour loger par déplacement longitudinal la tige de fixation (10) s'y raccordant essentiellement le long d'une colonne vertébrale et s'étendant depuis celle-ci en l'entourant sur plus de 180°, et un dispositif à vis (20.1, 20.2) avec un élément comportant un filetage externe (20.1) et un axe de filetage longitudinal (14) étant prévu pour serrer la tige de fixation (10), lequel dispositif à vis (20.1, 20.2) étant conçu et disposé de façon telle que les parois latérales (18.2, 18.3) de l'élément de bride (18) sont comprimées l'une vers l'autre pour serrer la tige de fixation (10),
**caractérisé en ce que**
l'écartement de l'axe longitudinal du filetage (14) au centre du cercle inscriptible dans la base de gouttière (18.1) correspond à la somme des rayons de ce cercle inscriptible et du boulon de vis (20.1) de préférence sans filetage dans cette zone.

2. Elément de bride (18) selon la revendication 1,
**caractérisé en ce que**
l'écartement mutuel des parois latérales (18.2, 18.3) est inférieur au diamètre du cercle inscriptible dans la base de bride (18.1).

3. Elément de bride (18) selon au moins l'une des revendications précédentes,
**caractérisé en ce que**
le dispositif à vis (20.1, 20.2) présente un filetage interne (24) dans une des parois latérales (18.3) et un filetage externe sur un boulon (20.1) pouvant être vissé dans le filetage interne (24) par un perçage traversant (22) pratiqué dans l'autre paroi latérale (18.2).

4. Elément de bride (18) selon au moins l'une des revendications précédentes,
**caractérisé en ce que**
la paroi latérale (18.3) pourvue du filetage interne (24) est conçue de façon plus renforcée dans la direction de l'axe (14) du dispositif à vis (20.1, 20.2) que la paroi latérale (18.2) pourvue du perçage traversant (22).

5. Elément de bride (18) selon au moins l'une des revendications précédentes,
**caractérisé en ce que**
la surface orientée vers l'extérieur de l'élément de bride (18) de la paroi latérale (18.2) équipée du perçage traversant (22) présente au moins une saillie (26) qui peut être déformée de façon élastique au moyen d'un écrou (20.2) ou d'une tête de vis du dispositif à vis (20.1, 20.2) pour former une sécurité de vissage.

6. Elément de bride (18) selon au moins l'une des revendications précédentes,
**caractérisé en ce que**
les parois latérales (18.2, 18.3) de l'élément de bride (18), dont l'écartement mutuel est inférieur au diamètre de la tige de fixation (10), peuvent être écartées l'une de l'autre de façon élastique avant l'insertion de la tige de fixation (10).

7. Crochet (12) présentant un élément de bride (18) pour serrer une tige de fixation (10) et un élément de crochet (16) formé de façon intégrale avec l'élément de bride (18) et dépassant d'une paroi latérale (18.3) de l'élément de bride (18), lequel est défini pour saisir par dessus ou dessous une partie dépassant latéralement (1) d'une colonne vertébrale,
**caractérisé en ce que**
l'élément de bride (18) est conçu selon au moins l'une des revendications 1 à 6.

8. Crochet (12) selon la revendication 6,
**caractérisé en ce que**
l'élément de crochet (16) est conçu de façon telle que le crochet (12) peut être utilisé en tant que crochet pédiculaire, laminaire, lombaire ou transversal.

9. Raccord de tige (32) présentant un élément de bride (18) pour serrer une tige de fixation (10) et un dispositif de serrage (34), formé de façon intégrale avec l'élément de bride (18), d'une tige transversale (10.1) s'étendant transversalement à la tige de fixation,
**caractérisé en ce que**
l'élément de bride (18) est conçu selon au moins l'une des revendications 1 à 6.

10. Raccord de tige selon la revendication 9,
**caractérisé en ce que**
le raccord de tige (32) y compris le dispositif à vis (20.1, 20.2) est fabriqué dans un matériau identique à celui des autres éléments du dispositif fixateur, de préférence dans un alliage de titane - niobium.
